(19)

Europäisches Patentamt
European Patent Office
Office européen des brevets

(11) **EP 4 524 969 A1**

(12) **EUROPEAN PATENT APPLICATION**
published in accordance with Art. 153(4) EPC

(43) Date of publication:
**19.03.2025 Bulletin 2025/12**

(51) International Patent Classification (IPC):
**G16B 5/00** (2019.01)

(21) Application number: 23940002.1

(52) Cooperative Patent Classification (CPC):
**G16B 5/00**

(22) Date of filing: **29.06.2023**

(86) International application number:
**PCT/JP2023/024250**

(87) International publication number:
**WO 2025/004283 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **FRONTEO, Inc.
Tokyo 108-0075 (JP)**

(72) Inventors:
• **TOYOSHIBA, Hiroyoshi
Tokyo 108-0075 (JP)**
• **TAKAGI, Yuna
Tokyo 108-0075 (JP)**

• **HAYASHI, Kazumi
Tokyo 108-0075 (JP)**
• **MIKAMI, Toshiyuki
Tokyo 108-0075 (JP)**
• **SAKAI, Miyuki
Tokyo 108-0075 (JP)**
• **MIYAMOTO, Makoto
Tokyo 108-0075 (JP)**
• **SAIJO, Eri
Tokyo 108-0075 (JP)**

(74) Representative: **Vossius & Partner
Patentanwälte Rechtsanwälte mbB
Siebertstrasse 3
81675 München (DE)**

(54) **PATHWAY GENERATION DEVICE, PATHWAY GENERATION METHOD, AND PATHWAY GENERATION PROGRAM**

(57) When a pathway is generated based on property information representing a property of a molecule acting on a disease or symptom, interactome information representing a connection relationship between molecules, and similarity information representing a similarity between molecules, the pathway is generated using a minimum flow algorithm to satisfy at least two conditions that (1) a path is generated by connecting only molecules whose flow values are greater than a minimum threshold and less than a maximum threshold, and (2) a path is generated by connecting only molecules whose flow values are equal to or less than a threshold related to a similarity. In this way, when a specific molecule is knocked out in a pathway once generated and the pathway is regenerated, changes occur not only in a path in which the knocked-out molecule is present but also in other paths, and an appropriate pathway reflecting the knocked-out state can be generated.

Fig. 4

THRESHOLD OF SIMILARITY=0. 4

(a)    (b)

**Description**

Technical Field

The present invention relates to a pathway generation apparatus, a pathway generation method, and a pathway generation program, and is particularly suitable for use in a technology for generating a pathway representing an intermolecular interaction as a route map.

Background Art

Conventionally, there has been a known pathway (also referred to as an intermolecular network) representing an intermolecular interaction as a route map. The pathway represents a molecule of a gene, protein, etc. using a symbol such as a circle or a square, and is expressed by connecting symbols with arrows that represent intermolecular interactions. Such visualization of the intermolecular interaction allows easier understanding of life phenomena such that it is possible to investigate which path contains a gene group whose expression level has changed. For example, a pathway is widely used in a field of disease treatment or drug discovery.

Note that there has been a known technology for generating a pathway by specifying a connection relationship between molecules using a minimum flow algorithm (for example, see PTL 1). In a pathway generation apparatus described in PTL 1, a pathway is generated by disposing a causality molecule on an upstream side and a responsiveness molecule on a downstream side, reflecting a connection relationship between molecules shown in a known knowledge database, and causing molecules having close similarity values between the disease feature vector and the molecule feature vector to be easily connected to each other. In this instance, a minimum flow algorithm is used to specify a sequential connection relationship among three or more molecules not shown in the known knowledge database.

In addition, there has been a known technology for generating an intermolecular network of any range according to an instruction of a user (for example, see PTL 2). As an example, PTL 2 discloses a method in which one or more molecules are designated in an intermolecular network generated once to perform connection search again, and the intermolecular network generated there (which becomes a partial network of an original intermolecular network) is marked and displayed. This method is considered to be useful for predicting the extent of the intermolecular network to be affected, for example, when expression of a certain protein is suppressed by a method such as knockout or RNA interference, or when a function of a certain protein is modified by a transgenic (also referred to as knock-in) method.

However, as illustrated in FIG. 12 of PTL 2, the technology described in PTL 2 merely obtains and marks a partial network that connects a molecule (indicated by one * in FIG. 12) designated by the user as a start point and a molecule (indicated by two #s in FIG. 12) designated by the user as an end point in an intermolecular network once generated through connection search (inverts and displays molecules belonging to the partial network). Therefore, for example, it has not been possible to analyze which effect occurs other than the partial network connecting the start point and the end point designated by the user when one certain molecule is knocked out or knocked in.

PTL 1: JP6915818B
PTL 2: JP2008-515029A

Summary of Invention

Technical Problem

The invention has been made to solve such a problem, and an object of the invention is to make it possible to analyze what effect occurs in the entire pathway when a specific molecule is knocked out in the pathway (intermolecular network) once generated.

Solution to Problem

To solve the above-mentioned problem, the invention generates a pathway representing an intermolecular interaction as a route map based on property information representing a property of a molecule acting on a disease or a symptom, connection information representing a connection relationship between molecules, and similarity information representing a similarity between molecules by disposing a causality molecule on an upstream side and a responsiveness molecule on a downstream side, reflecting a connection relationship indicated by the connection information, and causing molecules having high similarities indicated by the similarity information to be easily connected to each other. In this instance, a pathway is generated using a minimum flow algorithm in order to satisfy at least two conditions that (1) a path is generated by connecting only molecules whose flow values are greater than a minimum threshold and less than a maximum threshold, and (2) a path is generated by connecting only molecules whose flow values are equal to or less than a threshold related to a similarity.

Advantageous Effects of Invention

According to the invention configured as described above, not only a path passing through a molecule whose flow value is equal to or less than a minimum threshold or equal to or greater than a maximum threshold, but also a path connecting molecules having low similarities or a path passing through a molecule whose flow value exceeds a threshold related to a similarity are eliminated. As a result, the entire pathway (intermolecular network) is generated by selecting an intermolecular connection based on a flow value or the magnitude of a similarity from among intermolecular connections defined by connection information representing a connection relationship between molecules.

For this reason, when a specific molecule is knocked out in a pathway once generated and the pathway is regenerated, changes occur not only in a path in which the knocked-out molecule is present but also in other paths according to changes in a relationship between a redistributed flow value and a threshold, and an appropriate pathway reflecting the knocked-out state is generated. As described above, according to the invention, it is possible to analyze what effect occurs in the entire pathway when a specific molecule is knocked out in a pathway once generated.

Brief Description of Drawings

[FIG. 1] FIG. 1 is a block diagram illustrating a functional configuration example of a pathway generation apparatus according to this embodiment.
[FIG. 2] FIG. 2 is a block diagram illustrating a functional configuration example of a feature vector computation apparatus.
[FIG. 3] FIG. 3 is a diagram illustrating an example of a molecule feature vector.
[FIG. 4] FIG. 4 is a schematic diagram for describing conditions (1) and (2) when a minimum flow algorithm is executed.
[FIG. 5] FIG. 5 is a diagram illustrating an example of a pathway generation method.
[FIG. 6] FIG. 6 is a diagram illustrating an example of regenerating a pathway by knocking out one molecule included in a pathway generated once.
[FIG. 7] FIG. 7 is a block diagram illustrating another functional configuration example of the pathway generation apparatus according to this embodiment.

Description of Embodiments

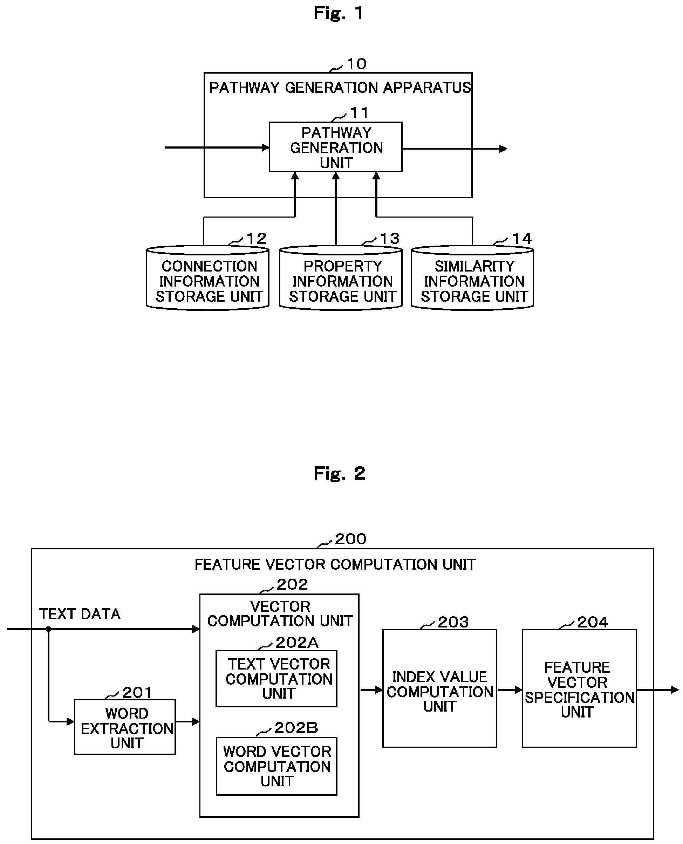

An embodiment of the invention will be described below with reference to the drawings. FIG. 1 is a block diagram illustrating a functional configuration example of a pathway generation apparatus 10 according to this embodiment. As illustrated in FIG. 1, the pathway generation apparatus 10 of this embodiment includes, as a functional configuration, a pathway generation unit 11. In addition, as storage media, a connection information storage unit 12, a property information storage unit 13, and a similarity information storage unit 14 are connected to the pathway generation apparatus 10 of this embodiment.

The pathway generation unit 11 can include any of hardware, a DSP (Digital Signal Processor), and software. For example, the pathway generation unit 11 is realized by an operation of a pathway generation program stored in a storage medium such as a RAM, a ROM, a hard disk, or a semiconductor memory under control of a microcomputer including a CPU, a RAM, a ROM, etc.

The connection information storage unit 12 stores connection information that indicates a connection relationship between molecules. The connection information includes known information such as interactome information. For example, the interactome information is known information related to an intermolecular interaction, such as when an expression level of a certain molecule (gene or protein) increases (or decreases), an expression level of another molecule increases (or decreases) in conjunction with the increase (or decrease) of the expression level of the certain molecule.

Note that this interactome information merely indicates which molecules are related to each other, and does not include information indicating magnitude of a connection relationship. In addition, the interactome information is a collection of information indicating a connection relationship between two molecules, and is not information indicating a sequential connection relationship among three or more molecules.

The property information storage unit 13 stores property information representing a property of a molecule acting on a disease or a symptom. A property of a molecular indicates whether the molecule acts causally or responsively on a disease or a symptom. Causality is a property considered to have a possibility of causing a disease or a symptom due to presence or mutation of the molecule. Responsiveness is a property considered to have a possibility that the molecule may mutate due to onset of a disease or symptom.

Here, when generating a pathway related to a specific disease (hereinafter referred to as a disease pathway), it is possible to use known information recorded in various literature, databases, etc. as information on a plurality of molecules related to the disease and information on properties (causality or responsiveness) of the molecules acting on the disease. In addition, it is possible to use information obtained by estimating a molecule related to a specific disease using a specified algorithm, and estimating a property of a specific molecule acting on the disease using a specified algorithm.

Any algorithm can be used as an algorithm for estimating a molecule related to a disease or for estimating a property of a molecule. For example, a new molecule related to a disease can be estimated by an estimation model trained by machine learning using known information on relevance between a disease and a molecule. In addition, a property of a new molecule can be estimated by an estimation model trained by machine learning using known information on a property of a molecule that acts on a disease.

This description is similarly applied to the case where a pathway related to a specific symptom (hereinafter referred to as a symptom pathway) is generated. That is, known information recorded in various literature, databases, etc. can be used as information on a plurality of molecules related to a symptom and information on properties of these molecules acting on the symptom. In addition, it is possible to use information obtained by estimating a molecule related to a specific symptom using a specified algorithm, and estimating a property of a specific molecule acting on the symptom using a specified algorithm.

The similarity information storage unit 14 stores similarity information representing a similarity between molecules. Here, the similarity information storage unit 14 stores information representing a similarity between respective molecules for a plurality of molecules stored as interactome information in the connection information storage unit 12. The similarity between molecules can be evaluated using various methods. For example, it is possible to apply a method of extracting a predetermined feature quantity for each piece of information related to a plurality of molecules (for example, a description of the molecules, structures or functions of the molecules, etc.) and evaluating a similarity of the feature quantity. As an example, it is possible to use a similarity between pairs of molecules expressed by a fingerprint method.

As another example, it is possible to compute a molecule feature vector by analyzing a plurality of texts (such as literature information) describing information related to a molecule, and use a similarity of the molecule feature vector. The molecule feature vector is data that represents features possessed by a molecule of protein, a gene, etc. (features that can identify the molecule) as a combination of values of a plurality of elements. As an example, a vector that represents a text to which a molecule name included as a word in a plurality of texts contributes and a degree to which the molecule name contributes to the text is used as the molecule feature vector.

A molecule name as a word tends to be used in a text having a description related to a molecule, and not used in a text unrelated to the molecule. In addition, among texts describing a molecule, a text in which a certain molecule name is included as a word is a text describing the molecule, and there is a high possibility that the molecule name is not included in a text describing another type of molecule. In other words, a text in which a molecule name is included as a word tends to vary depending on the type of molecule treated as a theme by the text. Therefore, a vector that represents a text to which a molecule name contributes and a degree to which the molecule name contributes to the text can be used as a feature vector that can identify a molecule.

Such a molecule feature vector can be computed using, for example, an algorithm described in JP6915818B. Hereinafter, a brief description will be given of a method of computing a molecule feature vector using the algorithm described in JP6915818B. FIG. 2 is a block diagram illustrating a functional configuration example of a feature vector computation apparatus 200.

In FIG. 2, a word extraction unit 201 analyzes m texts (m is any integer equal to or greater than 2) and extracts n words (n is any integer equal to or greater than 2) from the m texts. For example, known morphological analysis can be used as text analysis. Note that a plurality of same words may be included in the m texts. In this case, the word extraction unit 201 extracts only one word without extracting the plurality of same words. In other words, the n words extracted by the word extraction unit 201 mean n types of words.

A vector computation unit 202 computes m text vectors and n word vectors from m texts and n words. Here, a text vector computation unit 202A converts each of the m texts to be analyzed by the word extraction unit 201 into a q-dimensional vector according to a predetermined rule to compute m text vectors $d_i\rightarrow$ (i = 1, 2, ..., m) (symbol "$\rightarrow$" indicates being a vector) including q axis components (q is any integer equal to or greater than 2). In addition, a word vector computation unit 202B converts each of the n words extracted by the word extraction unit 201 into a q-dimensional vector according to a predetermined rule to compute n word vectors $w_j\rightarrow$ (j = 1, 2, ..., n) including q axis components. A specific method of computing the text vectors $d_i\rightarrow$ and the word vectors $w_j\rightarrow$ will not be described here.

An index value computation unit 203 computes each of the inner products of the m text vectors $d_i\rightarrow$ and the n word vectors $w_j\rightarrow$ computed by the vector computation unit 202, thereby computing index values reflecting a relationship between the m texts $d_i$ and the n words $w_j$. Here, as shown in the following Equation (1), the index value computation unit 203 obtains the product of a text matrix D having the respective q axis components ($d_{11}$ to $d_{mq}$) of the m text vectors $d_i\rightarrow$ as

respective elements and a word matrix W having the respective q axis components ($w_{11}$ to $w_{nq}$) of the n word vectors $w_j\rightarrow$ as respective elements, thereby computing an index value matrix DW having m × n index values as elements. Here, $W^t$ is the transposed matrix of the word matrix.

[Equation 1]

$$D = \begin{bmatrix} d_{11} & d_{12} & \cdots & d_{1q} \\ d_{21} & d_{22} & \cdots & d_{2q} \\ \cdot & \cdot & \cdot & \cdot \\ \cdot & \cdot & \cdot & \cdot \\ \cdot & \cdot & \cdot & \cdot \\ d_{m1} & d_{m2} & \cdots & d_{mq} \end{bmatrix} \qquad W = \begin{bmatrix} w_{11} & w_{12} & \cdots & w_{1q} \\ w_{21} & w_{22} & \cdots & w_{2q} \\ \cdot & \cdot & \cdot & \cdot \\ \cdot & \cdot & \cdot & \cdot \\ \cdot & \cdot & \cdot & \cdot \\ w_{n1} & w_{m2} & \cdots & w_{mq} \end{bmatrix}$$

$$DW = D * W^t = \begin{bmatrix} dw_{11} & dw_{12} & \cdots & dw_{1n} \\ dw_{21} & dw_{22} & \cdots & dw_{2n} \\ \cdot & \cdot & \cdot & \cdot \\ \cdot & \cdot & \cdot & \cdot \\ \cdot & \cdot & \cdot & \cdot \\ dw_{m1} & dw_{m2} & \cdots & dw_{mn} \end{bmatrix} \qquad \cdots (1)$$

Each element of the index value matrix DW computed in this manner may indicate which word contributes to which text and to what extent and which text contributes to which word and to what extent. For example, an element $dw_{12}$ in the first row and the second column may be a value indicating a degree at which the word $w_2$ contributes to a text $d_1$ and may be a value indicating a degree at which the text $d_1$ contributes to a word $w_2$. In this way, each row of the index value matrix DW can be used to evaluate a similarity of a text, and each column can be used to evaluate a similarity of a word.

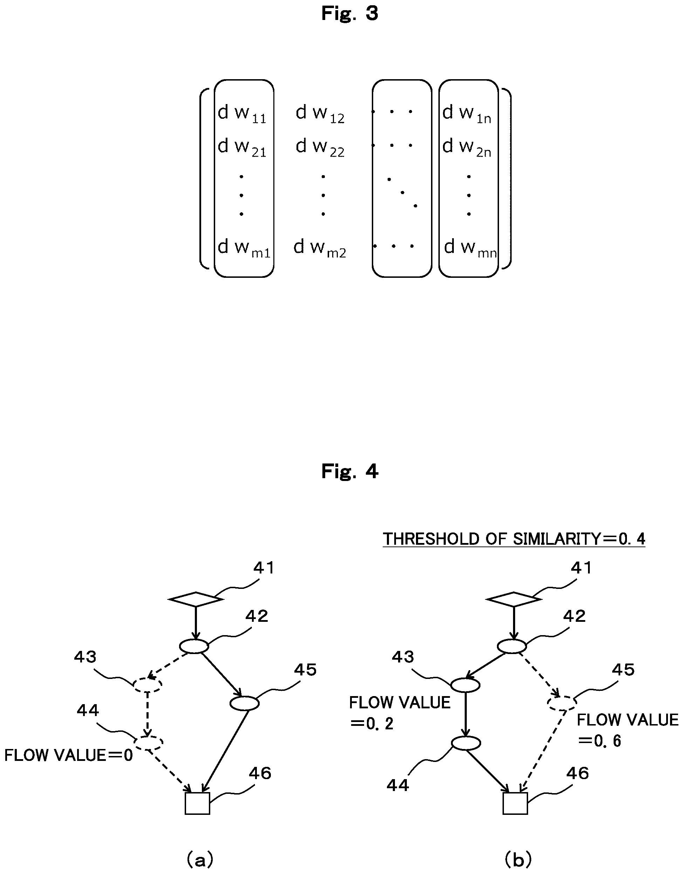

A feature vector specification unit 204 specifies, as a molecule feature vector, a word index value group including m index values for one molecule name for each of a plurality of molecule names among n words. That is, as illustrated in FIG. 3, the feature vector specification unit 204 specifies, as a molecule feature vector corresponding to each molecule name, a word index value group related to a word corresponding to a molecule name among n sets of word index value groups (m index values per column) constituting respective columns of the index value matrix DW.

A similarity of molecule feature vectors can be evaluated in various ways. For example, it is possible to apply a method of extracting a feature quantity for each molecule feature vector using a predetermined function, and evaluating a similarity of the feature quantity. Alternatively, a Euclidean distance or cosine similarity between molecule feature vectors may be used, or an edit distance may be used.

A description will be given by returning to FIG. 1. For example, the pathway generation unit 11 generates a pathway representing an intermolecular interaction as a route map through optimization processing using a minimum flow algorithm based on property information representing a property of a molecule acting on a specific disease or symptom designated by a user, connection information (interactome information) representing a connection relationship between molecules for the designated disease or symptom, and similarity information representing a similarity between molecules. The interactome information, the property information, and the similarity information are used by being read from the connection information storage unit 12, the property information storage unit 13, and the similarity information storage unit 14, respectively.

In this case, the pathway generation unit 11 generates a pathway with a molecule of causality (hereafter, causality molecule) indicated by the property information on an upstream side and a molecule of responsiveness (hereafter, responsiveness molecule) on a downstream side, and by reflecting a connection relationship indicated by the interactome information to place other molecules (hereafter referred to as connectivity molecules) between the causality molecule and the responsiveness molecule, and causing molecules having high similarities as indicated by similarity information to be more likely to be connected to each other. Here, the pathway generation unit 11 sets one start point on the further upstream side of all causality molecules, sets one end point on the further downstream side of all responsiveness molecules, and applies a minimum flow algorithm to a section from the start point to the end point.

Here, when a specific disease is designated by the user, the pathway generation unit 11 generates, as a disease pathway, an intermolecular network in which an intermolecular interaction of a molecule related to the designated disease is represented as a route map. In addition, when a specific symptom is designated by the user, the pathway generation unit 11 generates, as a symptom pathway, an intermolecular network in which an intermolecular interaction of a molecule related to the designated symptom is represented as a route map.

In addition, in this embodiment, the pathway generation unit 11 applies the minimum flow algorithm so as to satisfy at least the following two conditions.

(1) A path is generated by connecting only molecules whose flow values are greater than a minimum threshold and less than a maximum threshold.
(2) A path is generated by connecting only molecules whose flow values are equal to or less than a threshold related to a similarity.

Here, a flow value is an evaluation value computed for each molecule when connecting molecules using the minimum flow algorithm. This evaluation value is computed to satisfy the following conditions.

(A) A flow value of a start point set on an upstream side of the causality molecule is distributed to each of the causality molecule, the connectivity molecule, and the responsiveness molecule in sequence, and a flow value aggregated at an end point set on a downstream side of the responsiveness molecule is the same as the flow value of the start point. A flow value of each molecule is smaller than the flow values of the start point and the end point.
(B) The magnitude of the flow value of each molecule is determined taking into account a similarity between molecules. For example, the flow value is determined based on a predetermined function or calculation algorithm that uses a similarity as a variable. This function or calculation algorithm is designed so that, as the similarity increases, the flow value increases.
(C) The flow value is normalized to a value between 0 and 1. The magnitude of the similarity between molecules is also normalized to a value between 0 and 1. In this case, the flow value may be considered as the similarity between molecules.

With regard to condition (1), for example, a minimum threshold of the flow value can be set to "0". This means that a path including a molecule having a flow value of "0" is not adopted, and any of flow values of respective molecules included in a generated pathway does not become "0". The maximum threshold of the flow value in condition (1) may be different from or the same as a threshold related to a similarity in condition (2). When the values are the same, it means that the maximum threshold of the flow value in condition (1) is defined by the threshold related to the similarity (condition (2) is incorporated into condition (1)).

FIG. 4 is a schematic diagram for describing the above-mentioned conditions (1) and (2). In a path illustrated in FIG. 4, a diamond-shaped node represents a causality molecule, a rectangle-shaped node represents a responsiveness molecule, and an oval-shaped node represents a connectivity molecule (similarly applied to the other figures described below).

As illustrated in FIG. 4(a), it is assumed that two paths can be adopted by the interactome information as a path from a causality molecule 41 to a responsiveness molecule 46. That is, it is possible to adopt a first path of the causality molecule 41 → connectivity molecules 42, 43, and 44 → the responsiveness molecule 46, and a second path of the causality molecule 41 → connectivity molecules 42 and 45 → the responsiveness molecule 46. Here, for example, when a flow value of the connectivity molecule 44 is computed to be zero, the first path is eliminated and the second path is adopted.

In addition, as illustrated in FIG. 4(b), similarly to 4(a), in a situation where the first path and the second path can be adopted (where the flow value of the connectivity molecule 44 is $\neq$ 0), it is assumed that a flow value of one connectivity molecule 43 branching off from the connectivity molecule 42 is computed to be 0.2, and a flow value of the other connectivity molecule 45 is computed to be 0.6. Here, when a threshold related to similarity is 0.4, the flow value of the connectivity molecule 45 exceeds an upper limit of the similarity, and thus the second path is eliminated and the first path is adopted. Note that, when the flow values of both connectivity molecules 43 and 45 are smaller than 0.4, both the first and second paths are adopted.

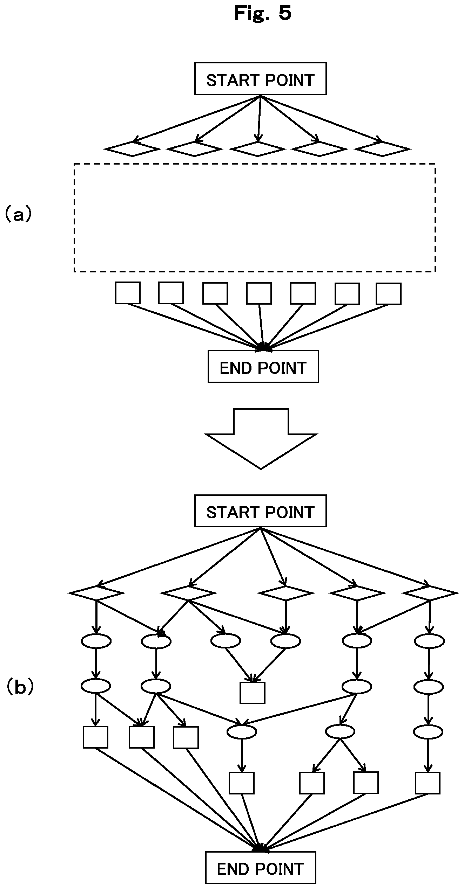

FIG. 5 is a diagram illustrating an example of a pathway generation method by the pathway generation unit 11. First, as illustrated in FIG. 5(a), the pathway generation unit 11 disposes the causality molecule on the upstream side, and disposes the responsiveness molecule on the downstream side. Then, a start point is set on the further upstream side of all causality molecules, and an end point is set on the further downstream side of all responsiveness molecules.

Next, as illustrated in FIG. 5(b), the pathway generation unit 11 applies a minimum flow algorithm to a section from the start point to the end point, and disposes connectivity molecules between causality molecules and responsiveness molecules based on a connection relationship indicated by interactome information, thereby generating a pathway. In this instance, the pathway generation unit 11 computes a flow value of each molecule to satisfy conditions (A) to (C), and performs optimization processing to satisfy conditions (1) and (2) based on the computed flow value.

In addition, when a specific molecule is designated to be knocked out from a pathway generated once, the pathway generation unit 11 regenerates the pathway using a minimum flow algorithm so that conditions (1) and (2) are satisfied in a state in which the molecule designated to be knocked out is deleted.

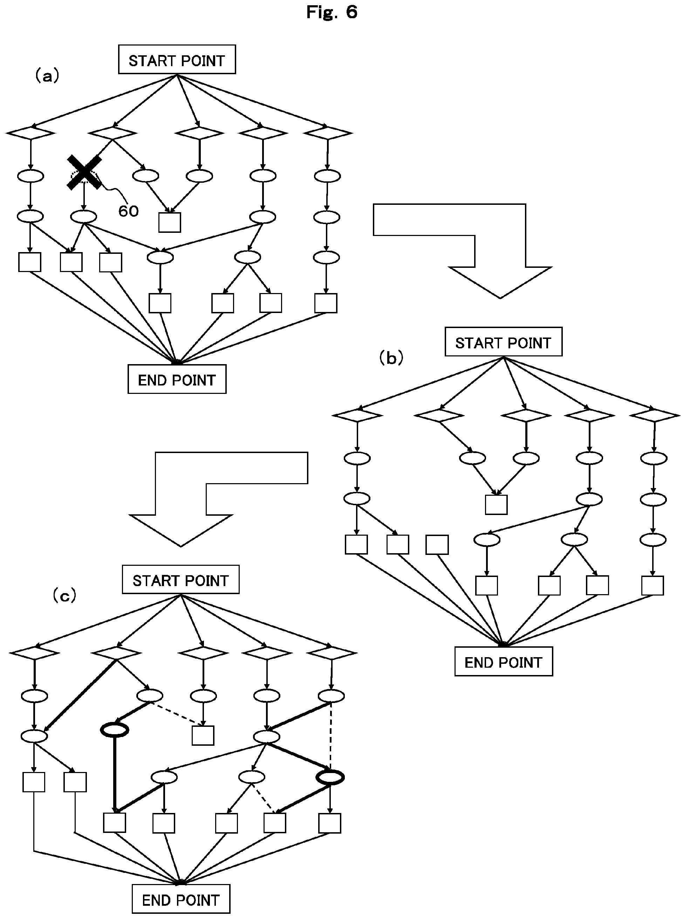

FIG. 6 is a diagram illustrating an example of regenerating a pathway by knocking out one connectivity molecule 60 included in a pathway generated once. FIG. 6(a) is a pathway generated as in FIG. 5(b), and illustrates that the one connectivity molecule 60 in this pathway is knocked out. A molecule to be knocked out can be arbitrarily designated. For example, it is possible to knock out a molecule that is a target gene for drug discovery.

Knocking out the molecule 60 is equivalent to forcibly setting the flow value of that molecule 60 to zero. Therefore, as illustrated in FIG. 6(b), a path passing through the knocked-out molecule 60 is eliminated. When a path including the knocked-out molecule 60 disappears, the flow value that has been assigned to the path is assigned to another path. In this instance, the pathway generation unit 11 recalculates a flow value of each molecule to satisfy conditions (A) to (C), and re-executes optimization processing based on the recalculated flow value to satisfy conditions (1) and (2).

Therefore, in addition to the path including the knocked-out molecule 60, a path that has been adopted before knockout since the path satisfied conditions (1) and (2) may disappear after knockout since the path no longer satisfies conditions (1) and (2). Conversely, a path that has not been adopted before knockout since the path does not satisfy conditions (1) and (2) may be adopted after knockout since the path satisfies conditions (1) and (2). As a result, the pathway before the knockout illustrated in FIG. 6(a) changes to a pathway illustrated in FIG. 6(c). In FIG. 6(c), a dotted line indicates a disappearing path, and a bold line indicates a newly created path.

As described above in detail, in this embodiment, when a pathway is generated based on property information representing a property of a molecule acting on a disease or a symptom, interactome information representing a connection relationship between molecules, and similarity information representing a similarity between molecules, the pathway is generated using a minimum flow algorithm in order to satisfy at least two conditions that (1) a path is generated by connecting only molecules whose flow values are greater than a minimum threshold and less than a maximum threshold, and (2) a path is generated by connecting only molecules whose flow values are equal to or less than a threshold related to a similarity.

According to this embodiment configured as described above, not only a path passing through a molecule whose flow value is equal to or less than a minimum threshold or equal to or greater than a maximum threshold, but also a path connecting molecules having low similarities or a path passing through a molecule whose flow value exceeds a threshold related to a similarity are eliminated. As a result, the entire pathway (intermolecular network) is generated by selecting an intermolecular connection based on a flow value or the magnitude of a similarity from among intermolecular connections defined by interactome information representing a connection relationship between molecules.

In this embodiment, since a similarity of a molecule feature vector computed from text data for a certain disease or symptom is used, an intermolecular connection based on the similarity of the molecule feature vector related to the designated disease or symptom is preferentially selected from among molecular connections defined by interactome information, and a pathway reflecting an intermolecular interaction related to the designated disease or symptom is generated.

That is, a similarity between molecules used in this embodiment indicates closeness between two molecule feature vectors obtained by analyzing a plurality of texts (literature information, etc.). By setting a threshold related to this similarity (for example, a threshold determined from the similarity) as an upper limit of a flow value that can pass between two molecules, in the case of a path that requires a certain amount or more of flow rate (an important path), a path between two molecules having low relevance is excluded by the flow rate limiting, and a path having high relevance is adopted. In other words, when the flow value exceeds the upper limit, processing is performed such that a molecule is replaced with a molecule having a higher similarity, or a flow value of another path is changed so that the upper limit is not exceeded. For this reason, molecules in which relevance between two molecules is high are selected in an important path, a connection between two molecules based on literature information on a certain disease is selected with priority, and a pathway close to a disease state is generated.

For this reason, when a specific molecule is knocked out in a pathway once generated and the pathway is regenerated, changes occur not only in a path in which the knocked-out molecule is present but also in other paths (including a path distant from the pathway in which the knocked-out molecule is present) according to changes in a relationship between a redistributed flow value and a threshold, and an appropriate pathway reflecting the knocked-out state is generated. As described above, according to this embodiment, it is possible to analyze what effect occurs in the entire pathway when a specific molecule is knocked out in a pathway once generated.

By utilizing this, for example, it is possible to predict an effect, resistance, and side effect of a drug against a specific disease. As an example, an analysis unit is further provided to analyze a difference between a pathway newly constructed by knocking out a specific molecule (gene) and a pathway before knockout, and it is possible to predict resistance to an anticancer drug using as an indicator that a gene changed in the pathway before and after knockout is a cancer-related gene (Cancer Hallmark). A pathway obtained by knocking out a specific gene is considered to be a pathway that exhibits resistance to an anticancer drug (treatment resistance) when anticancer drug treatment targeting the specific gene is performed.

Specifically, in this case, analysis is performed as follows. First, a disease pathway related to cancer to be analyzed is constructed, and a drug discovery target candidate gene X is selected. Next, the selected gene X is knocked out to construct a new disease pathway. Then, a constituent gene changed between two disease pathways is extracted, and a function of the constituent gene is analyzed. Here, it is analyzed whether the function of the changed gene corresponds to Cancer Hallmarks (an indicator that characterizes cancer). When the changed gene corresponds to Cancer Hallmarks, this indicates a possibility that new cancer may develop even if the target gene is inhibited, and resistance to the anticancer drug can be predicted.

Note that, in the above embodiment, in addition to conditions (1) and (2), one or both of the following conditions (3) and (4) may be added.

(3) A network size of the pathway falls within a preset range. The network size may be, for example, the number of molecules included on a path from a causality molecule to a responsiveness molecule, or the total number of molecules included in the entire pathway.

(4) When a plurality of paths can be adopted from one causality molecule to one responsiveness molecule, a shortest path (the number of included molecules is minimum) is selected therefrom. Alternatively, a path in which the sum of flow values of respective molecules from a causality molecule to a responsiveness molecule is maximum is adopted.

In addition, in the above embodiment, a description has been given on the assumption that the flow value of each molecule is a value determined by taking into account the similarity between molecules. However, the invention is not limited thereto. For example, the flow value of each molecule may be a value computed independently of the similarity between molecules, and the flow value of each molecule may be unequal to the similarity between molecules.

Furthermore, the disease pathway or symptom pathway may be generated using, for example, the algorithm described in JP6915818B.

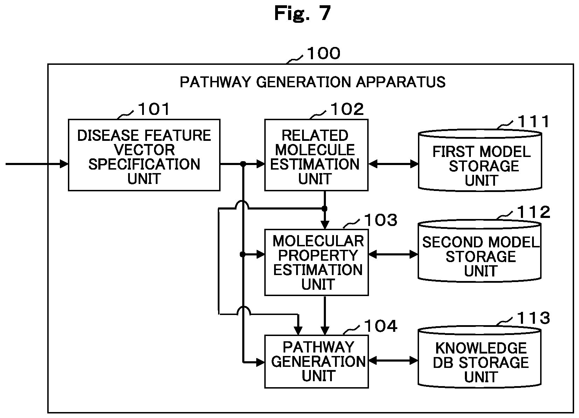

Hereinafter, a brief description will be given of a pathway generation method when using the algorithm described in JP6915818B. FIG. 7 is a block diagram illustrating a functional configuration example of a pathway generation apparatus 100 when the algorithm is used. Note that, here, the case where a disease pathway is generated will be described as an example.

A disease feature vector specification unit 101 specifies a feature vector (hereinafter referred to as a disease feature vector) corresponding to a disease name. The disease feature vector is data representing features of the disease (features that can identify the disease) as a combination of values of a plurality of elements. As an example, a vector representing a text to which a disease name included as a word in a plurality of texts contributes and a degree at which the disease name contributes to the text is used as a disease feature vector.

Such a disease feature vector can be computed, for example, by the feature vector computation apparatus 200 illustrated in FIG. 2. The feature vector computation apparatus 200 receives input of text data related to a text and computes a disease feature vector that reflects a relationship between the text and a word included therein. That is, in the index value matrix DW computed as illustrated in FIG. 3, among n word index value groups included in each column, a word index value group related to a word corresponding to a disease name is specified as a disease feature vector for each disease name.

A related molecule estimation unit 102 inputs a disease feature vector specified by the disease feature vector specification unit 101 to a first trained model stored in advance in a first model storage unit 111, thereby estimating a plurality of molecules associated with a disease. Here, the first trained model is subjected to machine learning so as to output information about a molecule corresponding to a molecule feature vector similar to a disease feature vector when the disease feature vector is input based on a similarity between the disease feature vector and the molecule feature vector.

The feature vector computation apparatus 200 computes a disease feature vector related to a plurality of disease names and computes a molecule feature vector related to a plurality of molecule names. Then, the first trained model is machine-trained in advance using these data sets, and the first trained model trained based on a similarity between the disease feature vector and the molecule feature vector is stored in the first model storage unit 111.

Here, the similarity between the disease feature vector and the molecule feature vector can be evaluated using various methods. For example, it is possible to apply a method of extracting a feature quantity using a predetermined function for each of the disease feature vector and the molecule feature vector and evaluating a similarity of the feature quantity. Alternatively, it is possible to use a Euclidean distance or cosine similarity between the word index value group of the disease feature vector and the word index value group of the molecule feature vector, or it is possible to use an edit distance.

A molecular property estimation unit 103 inputs a disease feature vector specified by the disease feature vector specification unit 101 and a molecule feature vector specified for a plurality of molecules estimated by the related molecule estimation unit 102 to a second trained model stored in a second model storage unit 112, thereby estimating a probability that a molecule acting on the disease is causative or responsive as a property for each of a plurality of molecules presumed to be associated with the disease.

Here, the second trained model is subjected to machine learning to output a probability that a property of a molecule is causative or responsive when a disease feature vector and a molecule feature vector are input using the disease feature vector, the molecule feature vector, and a data set of property information representing the property of the molecule acting on a disease as teacher data.

Using properties of molecules estimated by the molecular property estimation unit 103 and known interactome information indicating a connection relationship between molecules stored in a knowledge DB storage unit 113, a pathway generation unit 104 places a causality molecule on an upstream side and a responsiveness molecule on a downstream side for a plurality of molecules, relevance of which with respect to a disease is estimated by the related molecule estimation unit 102, and reflects a connection relationship indicated by interactome information in other connectivity molecules to dispose the connectivity molecules between the causality molecule and the responsiveness molecule, thereby generating a pathway (intermolecular network) representing an intermolecular interaction as a route map.

In this instance, for example, the pathway generation unit 104 computes a flow value of each molecule to satisfy conditions (A) to (C), and generates a pathway using a minimum flow algorithm to satisfy conditions (1) and (2) based on the computed flow value by disposing a causality molecule whose probability value estimated by the molecular property estimation unit 103 as causative is greater than a first threshold Th1 on an upstream side of the pathway, disposing a responsiveness molecule whose probability value is less than a second threshold Th2 (Th1 > Th2) on a downstream side of the pathway, and disposing a connectivity molecule whose probability value is greater than or equal to the second threshold Th2 and less than or equal to the first threshold Th1 between the causality molecule and the responsiveness molecule.

Here, as the similarity used in condition (B) when computing the flow value, for example, a value of a similarity between a disease feature vector specified by the related molecule estimation unit 102 and a molecule feature vector may be used. In addition, as a threshold related to the similarity used in condition (2), a threshold related to a similarity between a disease feature vector and a molecule feature vector may be used.

In addition, all the embodiments are merely examples of embodiment in carrying out the invention, and the technical scope of the invention should not be construed in a limited manner by the embodiments. That is, the invention can be implemented in various forms without departing from a gist or a main feature thereof.

Reference Signs List

    10, 100: pathway generation apparatus
    11: pathway generation unit
    12: connection information storage unit
    13: property information storage unit
    14: similarity information storage unit

**Claims**

1. A pathway generation apparatus comprising:

   a pathway generation unit configured to generate a pathway representing an intermolecular interaction as a route map based on property information representing a property of a molecule acting on a disease or a symptom, connection information representing a connection relationship between molecules, and similarity information representing a similarity between molecules by disposing a causality molecule indicated by the property information on an upstream side and a responsiveness molecule on a downstream side, reflecting a connection relationship indicated by the connection information, and causing molecules having high similarities indicated by the similarity information to be easily connected to each other,
   **characterized in that** the pathway generation unit generates the pathway using a minimum flow algorithm in order to satisfy at least two conditions that:

      (1) a path is generated by exclusively connecting molecules whose flow values are greater than a minimum threshold and less than a maximum threshold, and
      (2) a path is generated by exclusively connecting molecules whose flow values are equal to or less than a threshold related to the similarity.

2. The pathway generation apparatus according to claim **1, characterized in that** the pathway generation unit determines the flow value of each molecule based on the similarity between the molecules, and applies conditions (1) and (2) by setting a maximum threshold of the flow value and a threshold related to the similarity to be the same value.

3. The pathway generation apparatus according to claim 1 or 2, **characterized in that**, when a specific molecule is designated to be knocked out from a pathway generated once, the pathway generation unit regenerates the pathway using a minimum flow algorithm so that the at least two conditions are satisfied in a state in which the molecule designated to be knocked out is deleted.

4. The pathway generation apparatus according to claim **3, characterized by** further comprising an analysis unit configured to analyze a molecule changed from a pathway generated before deleting the specific molecule in a pathway regenerated in a state in which the specific molecule is deleted.

5. A pathway generation method comprising:

a process of generating, by a pathway generation unit of a computer, a pathway representing an intermolecular interaction as a route map based on property information representing a property of a molecule acting on a disease or a symptom, connection information representing a connection relationship between molecules, and similarity information representing a similarity between molecules by disposing a causality molecule indicated by the property information on an upstream side and a responsiveness molecule on a downstream side, reflecting a connection relationship indicated by the connection information, and causing molecules having high similarities indicated by the similarity information to be easily connected to each other, **characterized in that** the pathway generation unit generates the pathway using a minimum flow algorithm in order to satisfy at least two conditions that:

(1) a path is generated by exclusively connecting molecules whose flow values are greater than a minimum threshold and less than a maximum threshold, and
(2) a path is generated by exclusively connecting molecules whose flow values are equal to or less than a threshold related to the similarity.

6. A pathway generation program for causing a computer to function as:

a pathway generation means configured to generate a pathway representing an intermolecular interaction as a route map based on property information representing a property of a molecule acting on a disease or a symptom, connection information representing a connection relationship between molecules, and similarity information representing a similarity between molecules by disposing a causality molecule indicated by the property information on an upstream side and a responsiveness molecule on a downstream side, reflecting a connection relationship indicated by the connection information, and causing molecules having high similarities indicated by the similarity information to be easily connected to each other, **characterized in that** the pathway generation means generates the pathway using a minimum flow algorithm in order to satisfy at least two conditions that:

(1) a path is generated by exclusively connecting molecules whose flow values are greater than a minimum threshold and less than a maximum threshold, and
(2) a path is generated by exclusively connecting molecules whose flow values are equal to or less than a threshold related to the similarity.

**Fig. 1**

~10

PATHWAY GENERATION APPARATUS

~11

PATHWAY
GENERATION
UNIT

~12

CONNECTION
INFORMATION
STORAGE UNIT

~13

PROPERTY
INFORMATION
STORAGE UNIT

~14

SIMILARITY
INFORMATION
STORAGE UNIT

**Fig. 2**

~200

FEATURE VECTOR COMPUTATION UNIT

TEXT DATA

~202

VECTOR
COMPUTATION UNIT

~202A

TEXT VECTOR
COMPUTATION
UNIT

~202B

WORD VECTOR
COMPUTATION
UNIT

~201

WORD
EXTRACTION
UNIT

~203

INDEX VALUE
COMPUTATION
UNIT

~204

FEATURE
VECTOR
SPECIFICATION
UNIT

## Fig. 3

$$\begin{bmatrix} dw_{11} & dw_{12} & \cdots & dw_{1n} \\ dw_{21} & dw_{22} & \cdots & dw_{2n} \\ \vdots & \vdots & \ddots & \vdots \\ dw_{m1} & dw_{m2} & \cdots & dw_{mn} \end{bmatrix}$$

## Fig. 4

THRESHOLD OF SIMILARITY=0. 4

41
42
43
45
44
FLOW VALUE=0
46

(a)

41
42
43
45
FLOW VALUE
=0. 2
FLOW VALUE
=0. 6
44
46

(b)

# Fig. 5

## Fig. 6

(a)

(b)

(c)

## Fig. 7

PATHWAY GENERATION APPARATUS 100

DISEASE FEATURE VECTOR SPECIFICATION UNIT 101

RELATED MOLECULE ESTIMATION UNIT 102

FIRST MODEL STORAGE UNIT 111

MOLECULAR PROPERTY ESTIMATION UNIT 103

SECOND MODEL STORAGE UNIT 112

PATHWAY GENERATION UNIT 104

KNOWLEDGE DB STORAGE UNIT 113

## INTERNATIONAL SEARCH REPORT

| International application No. |
|---|
| **PCT/JP2023/024250** |

**A. CLASSIFICATION OF SUBJECT MATTER**

*G16B 5/00*(2019.01)i
FI: G16B5/00

According to International Patent Classification (IPC) or to both national classification and IPC

**B. FIELDS SEARCHED**

Minimum documentation searched (classification system followed by classification symbols)
G16B5/00-99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 6915818 B1 (FRONTEO INC.) 04 August 2021 (2021-08-04) entire text, all drawings | 1-6 |
| A | 長崎正朗ほか, "バイオパスウェイモデリングとシミュレーションを実現するためのシステム -Geonomic Object Net-", 人工知能学会誌, 01 January 2003, vol. 18, no. 1, pp. 8-13, ISSN:0912-8085, (NAGASAKI, Masao et al. Genomic Object Net: A Platform for Biopathway Modeling and Simulation. Journal of the Japanese Society for Artificial Intelligence.) entire text, all drawings | 1-6 |
| A | JP 2005-521929 A (GENOMATICA, INC.) 21 July 2005 (2005-07-21) entire text, all drawings | 1-6 |

☐ Further documents are listed in the continuation of Box C.    ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 July 2023** | **08 August 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2023/024250**

| Patent document cited in search report | | | Publication date (day/month/year) | Patent family member(s) | | | Publication date (day/month/year) |
|---|---|---|---|---|---|---|---|
| JP | 6915818 | B1 | 04 August 2021 | US | 2023/0122920 | A1 | |
| | | | | entire text, all drawings | | | |
| | | | | TW | 202203239 | A | |
| JP | 2005-521929 | A | 21 July 2005 | US | 2004/0029149 | A1 | |
| | | | | entire text, all drawings | | | |
| | | | | WO | 2003/082214 | A2 | |
| | | | | AU | 2003222128 | A1 | |
| | | | | CA | 2480216 | A1 | |
| | | | | EP | 1495321 | A2 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6915818 B

- JP 2008515029 A